# EUROPEAN PATENT SPECIFICATION

(11) **EP 3 081 181 B1**
(45) Date of publication and mention of the grant of the patent: **20.06.2018**
(21) Application number: 14852540.5
(22) Date of filing: 31.07.2014
(51) Int. Cl.: A61B 17/86, A61L 31/14

(54) **BIODEGRADABLE PURE MAGNESIUM BONE NAIL**
BIOLOGISCH ABBAUBARER KNOCHENNAGEL AUS REINEM MAGNESIUM
CLOU OSSEUX EN MAGNÉSIUM PUR BIODÉGRADABLE

(30) Priority: 10.10.2013 CN 201320625541 U
(43) Date of publication of application: 19.10.2016
(73) Proprietor: Dongguan Eontec Co., Ltd., Dongguan, Guangdong 523662 (CN)
(72) Inventor: LAO, Yonghua, Dongguan Guangdong 523662 (CN); LI, Yangde, Dongguan Guangdong 523662 (CN); LI, Weirong, Dongguan Guangdong 523662 (CN); LIU, Fangfei, Dongguan Guangdong 523662 (CN)
(74) Representative: Hryszkiewicz, Danuta
(86) International application number: PCT/CN2014/083364
(87) International publication number: WO 2015/051664

(56) References cited:
- EP-A1- 1 920 722
- WO-A1-2013/021913
- CN-A- 102 068 306
- CN-A- 102 247 622
- CN-A- 103 263 697
- CN-Y- 201 286 766
- US-A1- 2004 073 221
- US-A1- 2008 039 846

## Description

The invention relates to an orthopedic device and in particular to a biodegradable bone nail.

Conventional bone nails are made of stainless steel or titanium alloy. The two nails are not degraded in the body and may need to be removed after fracture healing. This increases pain and cost, and can lead to complications.

Magnesium alloy is an absorbable implant material, but the components thereof are complex, often resulting in cytotoxicity and adverse biological reactions, as shown in CN 102068306 A. Moreover, the magnesium-alloy bone nail disclosed in CN 102068306 A includes an axial plug hole and a plurality of non-axial through holes communicating with the axial plug hole; and when the bone nail is being inserted in the patient's body, the bone nail is hollow and correspondingly has a relatively low torsional strength.

In view of the above-described problems, it is one objective of the invention to provide a biodegradable bone nail that has simple structure and enhanced torsional strength, and causes no cytotoxicity and no adverse biological reactions.

To achieve the above objective, in accordance with one embodiment of the invention, there is provided a biodegradable bone nail, comprising a rod-shaped magnesium screw having a head comprising a cross groove, the magnesium screw comprising an axial chamber disposed below the cross groove, a depth of the axial chamber being two thirds that of the bone nail. Along a length direction of the axial chamber, a plurality of radial holes are equidistantly disposed every 90 degrees from bottoms of threads on a wall of the magnesium screw, and the radial holes communicate with the axial chamber.

The biodegradable bone nail is made of pure magnesium having a purity of between 99.9% and 99.99%. As a fixed implantable apparatus for bone repair and reconstruction, the biodegradable bone nail causes no cytotoxicity and no adverse biological reaction. When the biodegradable bone nail
is used for bone repair and reconstruction, prior to implanting, the axial chamber of the bone nail is filled with patient's own bone flap or cortical bone. The bone flap or cortical bone is compacted. The compacted bone flap or cortical bone significantly enhances the torsional strength of the bone nail and ensure the uniform stress of the bone nail during being implanted. In addition, patient's own bone flap or cortical bone filled in the axial chamber communicates with the bone tissues outside the bone nail through the radial holes to induce the growth of the bone, thus facilitating bone healing.

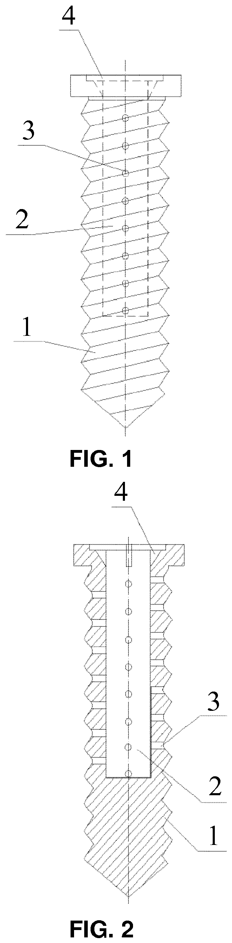

FIG. **1** is a schematic diagram of a biodegradable bone nail in accordance with one embodiment of the invention; and
FIG. **2** is a sectional view of a biodegradable bone nail in accordance with one embodiment of the invention.

In the drawings, the following number references are used: **1.** Bone nail; **2.** Axial chamber; **3.** Radial hole; **4.** Cross groove.

For further illustrating the invention, experiments detailing a biodegradable bone nail are described below. It should be noted that the following examples are intended to describe and not to limit the invention.

A biodegradable bone nail **1** comprises a rod-shaped magnesium screw having a head comprising a cross groove **4.** The magnesium screw comprises an axial chamber **2** disposed below the cross groove **4,** a depth of the axial chamber being two thirds that of the bone nail. Along a length direction of the axial chamber, a plurality of radial holes **3** are equidistantly disposed every 90 degrees from bottoms of threads on a wall of the magnesium screw, and the radial holes **3** communicate with the axial chamber **2.**

The biodegradable bone nail is made of pure magnesium having a purity of between 99.9% and 99.99%. As a fixed implantable apparatus for bone repair and reconstruction, the biodegradable bone nail causes no cytotoxicity and no adverse biological reactions. When the biodegradable
bone nail is used for bone repair and reconstruction, prior to implanting, the axial chamber **2** of the bone nail **1** is filled with patient's own bone flap or cortical bone. The bone flap or cortical bone is compacted. The compacted bone flap or cortical bone can significantly enhance the torsional strength of the bone nail **1** and ensure the uniform stress of the bone nail **1** during implantation. In addition, the bone flap or cortical bone filled in the axial chamber **2** can communicate with the bone tissues outside the bone nail through the radial holes **3** to induce the growth of the bone, thus facilitating the bone healing.

The biodegradable bone nail is made of pure magnesium having a purity of between 99.9% and 99.99%. As a fixed implantable apparatus for bone repair and reconstruction, the biodegradable bone nail causes no cytotoxicity and no adverse biological reactions.

## Claims

1. A biodegradable bone nail for being inserted into a patient's body, comprising a rod-shaped magnesium screw; the rod-shaped magnesium screw comprising a head having a cross groove, an axial chamber, and a plurality of radial holes;
**characterized in that:**
the axial chamber extends inwards from the cross groove to a two-thirds depth of the biodegradable bone nail;
the plurality of radial holes is disposed along the longitudinal direction of the axial chamber;
the plurality of radial holes communicates with the axial chamber;
the plurality of radial holes is equidistantly disposed every 90 degrees from bottoms of threads on a wall of the rod-shaped magnesium screw; and
the axial chamber is filled with the patient's bone flap or cortical bone.

## Patentansprüche

1. Ein biologisch abbaubarer Knochennagel, der in den Körper eines Patienten eingesetzt werden soll, umfassend eine stabförmige Magnesiumschraube; wobei die stabförmige Magnesiumschraube einen Kopf umfasst, der eine Querrille, eine axiale Kammer und eine Vielzahl von radialen Löchern aufweist;
**dadurch gekennzeichnet, dass:**
die axiale Kammer sich einwärts von der Querrille bis zu zwei Drittel der Tiefe des biologisch abbaubaren Knochennagels erstreckt;
die Vielzahl der radialen Löcher in der Längsrichtung der axialen Kammer angeordnet sind;
die Vielzahl der radialen Löcher mit der axialen Kammer in Verbindung steht;
die Vielzahl der radialen Löcher in gleichem Abstand alle 90 Grad ab Grund der Gewinde auf einer Wand der stabförmigen Magnesiumschraube angeordnet ist; und
die axiale Kammer mit dem Knochendeckel oder dem kortikalen Knochen des Patienten gefüllt ist.

## Revendications

1. Clou osseux biodégradable destiné à être inséré dans le corps d'un patient, comprenant une vis en magnésium en forme de tige ; la vis en magnésium en forme de tige comprenant une tête ayant une rainure transversale, une chambre axiale et une pluralité de trous radiaux ;
**caractérisé en ce que** :
la chambre axiale s'étend vers l'intérieur à partir de la rainure transversale jusqu'à une profondeur de deux tiers du clou osseux biodégradable ;
la pluralité de trous radiaux est disposée le long de la direction longitudinale de la chambre axiale ;
la pluralité de trous radiaux communique avec la chambre axiale ;
la pluralité de trous radiaux est disposée de manière équidistante tous les 90 degrés à partir du fond des filetages sur une paroi de la vis en magnésium en forme de tige ; et
la chambre axiale est remplie avec le volet osseux ou l'os cortical du patient.
